# EUROPEAN PATENT SPECIFICATION

(11) **EP 4 460 275 B1**
(45) Date of publication and mention of the grant of the patent: **23.09.2026**
(21) Application number: 22826843.9
(22) Date of filing: 13.12.2022
(51) Int. Cl.: A61F 9/007, A61M 1/00

(54) **PHACOEMULSIFIER WITH HERMETIC PROTECTION AGAINST DISTALLY-PROPAGATING PRESSURE PULSES**
PHACOEMULGATOR MIT HERMETISCHEM SCHUTZ GEGEN DISTAL SICH AUSBREITENDE DRUCKIMPULSE
PHACOÉMULSIFIANT À PROTECTION HERMÉTIQUE CONTRE DES IMPULSIONS DE PRESSION SE PROPAGEANT DISTALEMENT

(30) Priority: 07.01.2022 US 202217570964
(43) Date of publication of application: 13.11.2024
(73) Proprietor: Johnson & Johnson Surgical Vision, Inc., Irvine, CA 92618 (US)
(72) Inventor: GOVARI, Assaf, 2066717 Yokneam (IL); GLINER, Vadim, 2066717 Yokneam (IL); AHARON, Eran, 2066717 Yokneam (IL)
(74) Representative: Carpmaels & Ransford LLP
(86) International application number: PCT/IB2022/062122
(87) International publication number: WO 2023/131840

(56) References cited:
- WO-A1-2020/160434
- US-A1- 2015 359 666

## Description

### FIELD OF THE DISCLOSURE

The present disclosure relates generally to phacoemulsification systems, and particularly to methods and systems for protection against distally-propagating pressure pulses.

### BACKGROUND OF THE DISCLOSURE

A cataract is a clouding and hardening of the eye's natural lens, a structure which is positioned behind the cornea, iris and pupil. The lens is mostly made up of water and protein and as people age these proteins change and may begin to clump together obscuring portions of the lens. To correct this, a physician may recommend phacoemulsification cataract surgery. In the procedure, the surgeon makes a small incision in the sclera or cornea of the eye. Then a portion of the anterior surface of the lens capsule is removed to gain access to the cataract. The surgeon then uses a phacoemulsification probe, which has an ultrasonic handpiece with a needle. The tip of the needle vibrates at ultrasonic frequency to sculpt and emulsify the cataract while a pump aspirates particles and fluid from the eye through the tip. Aspirated fluids are replaced with irrigation of a balanced salt solution to maintain the anterior chamber of the eye. After removing the cataract with phacoemulsification, the softer outer lens cortex is removed with suction. An intraocular lens (IOL) is then introduced into the empty lens capsule restoring the patient's vision.

Various techniques of irrigation and aspiration control with medical probes were proposed in the patent literature. For example, U.S. Patent Application Publication 2006/0135974 describes a length of a tubing that includes an irrigation lumen for carrying irrigation fluid from a source to an ophthalmic surgical site at an eye. An aspiration lumen is also formed in a tubing for carrying aspirant from the surgical site at the eye to a collection reservoir. The irrigation lumen and the aspiration lumen include a compliant common wall. Wall ensures that any surge occurring after an occlusion break during surgery is dampened because of the compliant common wall.

As another example, U.S Patent 5,476,448 describes a surge suppresser which has a collapsible flexible wall that accumulates the aspiration fluid of an interocular surgical system. The surge suppresser has an inlet port and an outlet port that are connected to an aspiration line downstream from a surgical tip. The aspiration line is connected to a vacuum device which draws fluid from the tip. When an occlusion in the aspiration line occurs, the decrease in pressure caused by the vacuum device will cause the flexible wall to collapse and close the inlet and outlet ports of the surge suppresser. When the occlusion breaks, the inlet port initially opens and the outlet port remains closed so that the flexible wall is expanded by the flow of fluid from the surgical tip. The closed outlet port prevents a surge of fluid from the eye. Additionally, the ports are arranged in a parallel relationship to reduce the momentum of the fluid into the suppresser and further limit the surge of fluid from the eye. As the flexible wall gradually expands, the outlet port opens and allows the fluid to flow through the suppresser.

US 2015/0359666 A1 provides a cyclic aperture flow regulator system is to control flow exiting from a body cavity during Surgery in a way that post-occlusion surges are effectively suppressed. The system is composed by an adjustable fluid aperture installed in a fluid path connecting the aspiration port of a surgical probe with a vacuum source, the probe to be inserted in a body cavity. The cross-sectional area of the fluid aperture can be modified by the action of an actuator portion driven by a controller.

### SUMMARY

The invention provides a phacoemulsification system according to claim 1 and a method for producing a phacoemulsification system according to claim 10. Embodiments are provided by the dependent claims.

The present disclosure will be more fully understood from the following detailed description of the examples thereof, taken together with the drawings in which:

### BRIEF DESCRIPTION OF THE DRAWINGS

Fig. 1 is a schematic, pictorial view of a phacoemulsification system, in accordance with an example of the present disclosure;
Fig. 2 is a diagram that schematically illustrates part of a phacoemulsification system, showing a protection valve for protection against distally-propagating pressure pulses, in accordance with an example of the present disclosure; and
Fig. 3 is a flow chart that schematically illustrates a method for phacoemulsification, in accordance with an example of the present disclosure.

### DETAILED DESCRIPTION OF EXAMPLES

### OVERVIEW

During phacoemulsification of a cataracted lens of an eye, emulsified lens particles are aspirated via an aspiration line that runs through the phacoemulsification handpiece and further proximally to an aspiration pump. When a particle blocks the inlet of the aspiration line, the vacuum in the line increases. When the line later becomes unblocked (e.g., when the particle is subsequently sucked into the aspiration line), the high vacuum in the line resulting from the blockage causes an aspiration surge with potentially traumatic consequences to the eye.

When a vacuum surge is detected, a possible responsive measure is to close the vacuum line with a valve. However, vacuum buildup downstream of a closed valve may be accompanied, after the valve is reopened, with a pressure pulse that propagates toward the eye. The pressure pulse leads to flow into the eye. A typical pressure pulse in an aspiration line is extremely narrow in time, typically lasting several tens of milliseconds. If such a pressure pulse feeds back to the eye, it can cause trauma. The pulse may also cause material that is held by the tip of the handpiece to be released and/or repelled from the tip.

Examples of the present disclosure that are described herein provide methods and devices for protection against distally-propagating pressure pulses. In the disclosed examples, an additional valve is inserted in the aspiration line, e.g., in the handle of the phacoemulsification handpiece, for protection against distally-propagating pressure pulses. This additional valve is referred to herein as a "protection valve" in order to distinguish from other valves that may be used in the system.

In some examples, the protection valve is a piston-type valve, comprising a piston that moves in a chamber (also sometimes referred to as "cylinder"). The chamber has an inlet for receiving the evacuated material arriving from the phacoemulsification needle, and an outlet for flowing the material along the aspiration line. The piston is configured to controllably move in the chamber between at least (i) a first ("open") position that enables flow of material between the inlet and the outlet and (ii) a second ("closed") position that blocks the flow between the inlet and the outlet. In a typical example the protection valve is magnetically actuated, i.e., comprises an electromagnet that is configured to move the piston between the "open" and "closed" positions in response to a control signal.

In practice, however, the piston may not seal the chamber hermetically, even in the "closed" position. For example, a finite gap may remain between the piston and the chamber wall in order to enable the piston to move, and this gap may degrade the sealing quality. When sealing is imperfect, a pressure pulse hitting the protection valve may still propagate to the eye and cause damage.

To address this challenge, in some examples the protection valve further comprises a compressible seal that is attached to the inlet. The seal is designed specifically to block the inlet hermetically, when the piston is in the second ("closed") position and a pressure pulse impinges on the protection valve. The seal is designed to compress between the piston and the inlet, as a result of the force exerted on the piston by the pressure pulse.

In this manner, the compressed seal closes the inlet hermetically and prevents any residues of the pressure pulse from reaching the eye. After the pressure pulse is over, the pressure on the piston reduces, the seal relaxes and decompresses, enabling the piston to move freely in the chamber.

The seal may be implemented and attached to the inlet in various ways. For example, the seal may comprise sealing material that is overmolded over the circumference of the inlet. Alternatively, the seal may comprise an O-ring placed over the inlet circumference.

The disclosed protection valves provide simple yet effective protection against pressure pulses that may propagate in the aspiration line toward the eye. The description that follows refers mainly to pressure pulses that are caused by relief of vacuum buildup in the aspiration line following recovery from occlusion. The disclosed protection valves are useful, however, for protecting against various other types of pressure pulses or variations, e.g., pulses caused by peristaltic vacuum pumps.

### SYSTEM DESCRIPTION

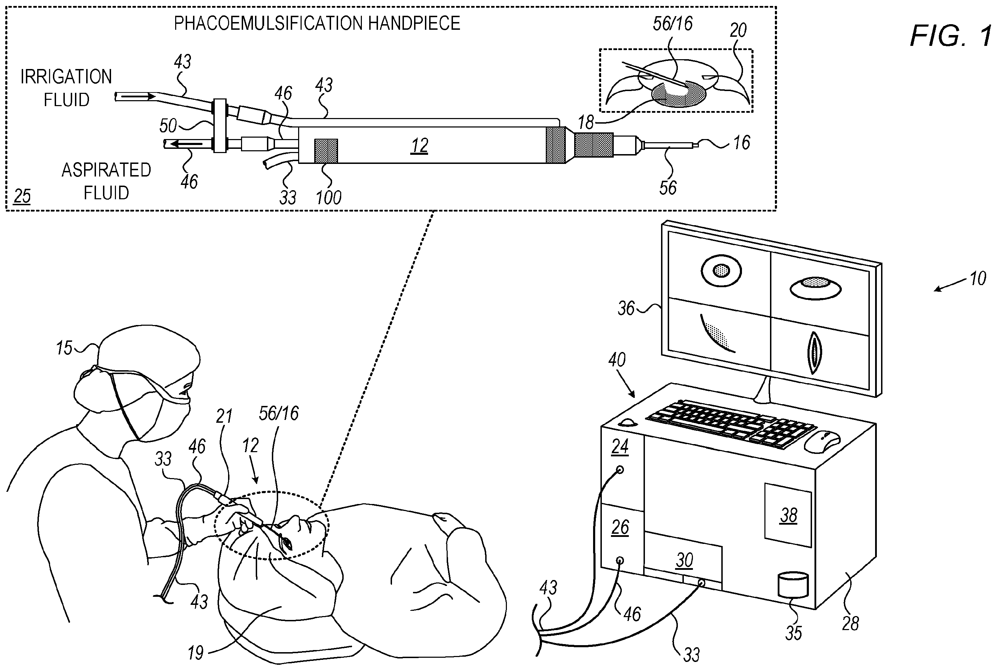

Fig. 1 is a schematic, pictorial view of a phacoemulsification system 10 comprising a phacoemulsification probe ("handpiece") 12, in accordance with an example of the present disclosure. An inset 25 illustrates the structure of the handpiece in greater detail.

Phacoemulsification probe 12 comprises a hollow needle 16, and a coaxial irrigation sleeve 56 that at least partially surrounds needle 16 and creates a fluid pathway between the external wall of the needle and the internal wall of the irrigation sleeve. Needle 16 is hollow, so as to provide an aspiration pathway. The irrigation sleeve may have one or more side ports at or near its distal end, to allow irrigation fluid to flow toward the distal end of the handpiece through the fluid pathway and out of the port(s).

Needle 16 is configured for insertion into a lens capsule 18 of an eye of a patient 19 by a physician 15, for treating a cataract. While needle 16 (and irrigation sleeve 56) are shown in inset 25 as a straight object, any suitable needle shape may be used with phacoemulsification probe 12, for example a curved or bent tip needle commercially available from Johnson & Johnson Surgical Vision, Inc. (Santa Ana, CA, USA).

In the shown example, during the phacoemulsification procedure a pumping subsystem 24 comprised in a console 28 pumps irrigation fluid from an irrigation reservoir to irrigation sleeve 56 to irrigate the eye. The irrigation fluid is pumped via an irrigation tubing line 43 running from the console 28. The distal section of irrigation line 43 passes via handpiece 12 to irrigation sleeve 56. Eye fluid and waste matter (e.g., emulsified parts of the cataract) are aspirated via hollow needle 16 to a collection receptacle (not shown) by a pumping subsystem 26, also comprised in console 28, using an aspiration tubing line 46 running from probe 12 to console 28. In another example, the pumping subsystem 24 may be coupled or replaced with a gravity-fed irrigation source such as a balanced salt solution bottle/bag.

In some examples, system 10 comprises an Anti-Vacuum Surge (AVS) module 50, coupled via suitable fluid connectors to the irrigation and aspiration lines. The AVS module is configured to control aspiration and irrigation flow rates, e.g., in order to reduce risks to the eye from irregular performance of aspiration and/or irrigation in probe 12, such as from vacuum surges. In some examples, the AVS module is coupled with the aspiration and irrigation lines externally to handpiece 12. In alternative examples the AVS module may be integrated in handpiece 12. Further aspects AVS modules are described in U.S. Patent Application 17/130,409, filed December 22, 2020, entitled "A module for Aspiration and Irrigation Control." The application discloses an anti-vacuum surge (AVS) module coupled to a phacoemulsification probe, which uses valves to prevent a sudden vacuum increase from being transferred into the eye when an occlusion breaks.

In an example, the AVS module establishes variable fluid communication between aspiration line 46 and irrigation line 43, to control the flow of fluid between the two lines, so as to maintain pressures in the two lines within predefined limits. Moreover, the AVS module can discontinue aspiration, in order to provide a fast response (e.g., within several milliseconds) to a detected occlusion or vacuum surge. The AVS module may comprise its own processor and can be used with existing phacoemulsification systems as a disposable element to improve control over intraocular pressure (IOP) during the surgical cataract removal procedure. In some examples, a different type of AVS module can be used that is coupled only to the aspiration part of the system (i.e., without involving irrigation).

Phacoemulsification probe 12 includes other elements (not shown), such as a piezoelectric crystal coupled to a horn to drive vibration of needle 16. The piezoelectric crystal may be configured to vibrate needle 16 in a resonant vibration mode. The vibration of needle 16 is used to break the cataract into small pieces during the phacoemulsification procedure. Console 28 comprises a piezoelectric drive module 30, coupled with the piezoelectric crystal, using electrical wiring running in a cable 33. Drive module 30 is controlled by a processor 38 and conveys processor-controlled driving signals via cable 33 to, for example, maintain needle 16 at maximal vibration amplitude. The drive module may be realized in hardware or software, for example, in a proportional-integral-derivative (PID) control architecture.

Processor 38 may receive user-based commands via a user interface 40, which may include setting a vibration mode, duty cycle, and/or frequency of the piezoelectric crystal, and setting or adjusting an irrigation and/or aspiration rate of the pumping subsystems 24/26. In an example, user interface 40 and display 36 may be combined as a single touch screen graphical user interface. In an example, the physician uses a foot pedal (not shown) as a means of control. Additionally or alternatively, processor 38 may receive the user commands from controls located in a handle 21 of probe 12.

Some or all of the functions of processor 38 may be combined in a single physical component or, alternatively, implemented using multiple physical components. These physical components may comprise hard-wired or programmable devices, or a combination of the two. In some examples, at least some of the functions of processor 38 may be carried out by suitable software stored in a memory 35 (as shown in Fig. 1). This software may be downloaded to a device in electronic form, over a network, for example. Alternatively or additionally, the software may be stored in tangible, non-transitory computer-readable storage media, such as optical, magnetic, or electronic memory.

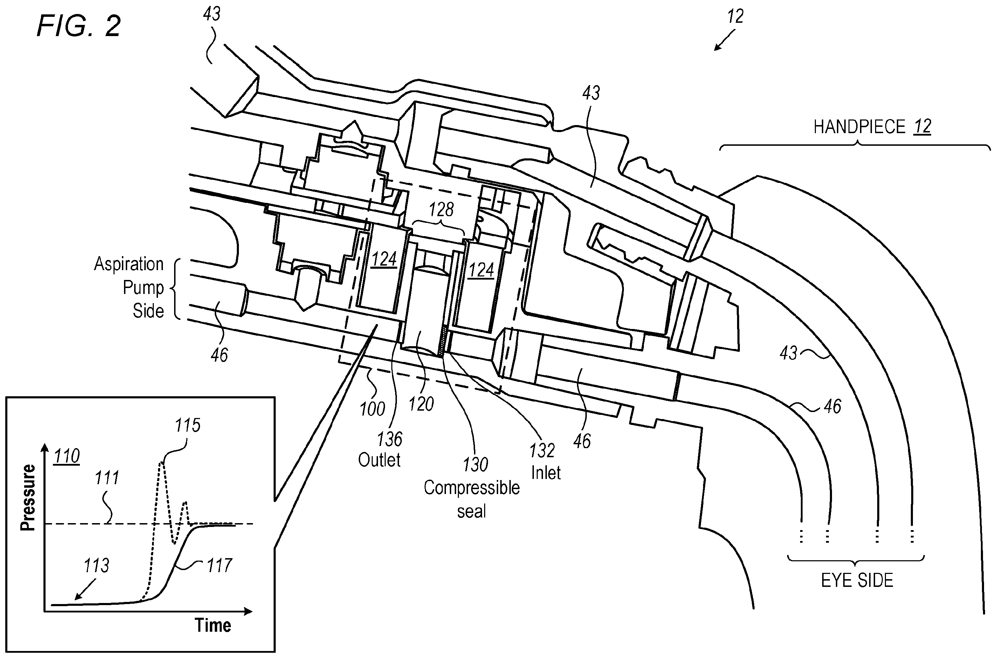

In the examples described herein, system 10 further comprises a protection valve 100, which is inserted in aspiration line 46 and is actuated by processor 38. In the example of Fig. 1 protection valve 100 is integrated in handpiece 12. Alternatively, protection valve 100 may be external handpiece 12. An example of an external protection valve is shown in Fig. 2 below.

Protection valve 100 is configured to block the aspiration line hermetically during distally-propagating pressure pulses. Such pulses may develop in the aspiration line, for example, following re-opening of a valve in an AVS module. As explained above, it is highly important to prevent such pressure pulses from reaching the eye. The structure and operation of protection valve 100 are addressed in detail below.

The system shown in Fig. 1 may include further elements which are omitted for clarity of presentation. For example, physician 15 typically performs the procedure using a stereomicroscope or magnifying glasses, neither of which are shown. Physician 15 may use other surgical tools in addition to probe 12, which are also not shown in order to maintain clarity and simplicity of presentation.

### PROTECTION AGAINST PRESSURE PULSES USING HERMETIC PROTECTION VALVE

Fig. 2 is a diagram that schematically illustrates part of system 10, showing protection valve 100, in accordance with another example of the present disclosure. Irrigation line 43 is seen at the top of the figure, and the aspiration line 46 is seen at the bottom. In the present example, the direction of aspiration is from right (labelled "eye side") to left (labelled "aspiration pump side"). Unlike the example of Fig. 1, in the present example protection valve 100 is external to handpiece 12. As seen in Fig. 2, protection valve 100 is inserted in aspiration line 46, just proximally to handpiece 12. The internal structure and operation of valve 100 are similar in both examples.

As seen, protection valve 100 comprises a piston 120, a chamber 128 and an electromagnet 124. Chamber 128 has an inlet 132 for receiving material arriving from the eye side, and an outlet 136 for flowing the material onwards toward the aspiration pump. Electromagnet 124 is actuated by a control signal from processor 38. Using the control signal, processor 38 controls electromagnet 124 to move piston 120 between an "open" position and a "closed" position.

In the closed position (which is the position shown in the figure) piston 120 blocks the flow of material between inlet 132 and outlet 136. In the open position the piston moves away from the aspiration line (moves upwards, according to the orientation of the figure) and enables flow of material between the inlet and the outlet.

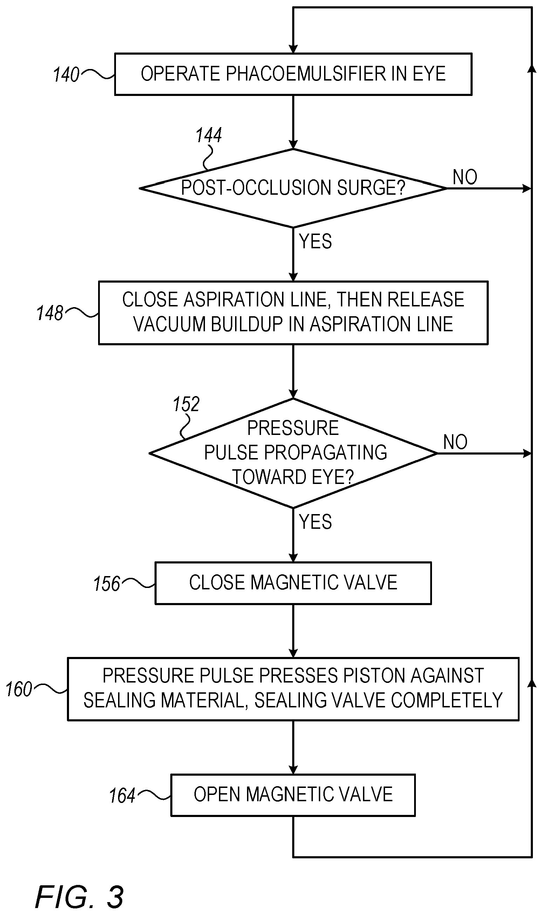

In some examples, processor 38 closes protection valve 100 in response to detecting that a distally-propagating pressure pulse is imminent. An example method of this sort is depicted in Fig. 3 below.

In practice, even when piston 120 is in the "closed" position, the flow of material through chamber 128 may not be blocked completely. For example, some finite gap may still remain between piston 120 and inlet 132, and/or between piston 120 and outlet 136, in order to allow the piston to move freely between the "open" and "closed" positions. Such imperfect sealing may degrade the performance of protection valve 100 in protecting the eye from pressure pulses.

In some examples, protection valve 100 further comprises a compressible seal 130 that provides hermetic sealing of the valve during a pressure pulse. Consider a scenario in which a distally-propagating pressure pulse impinges on protection valve 100, e.g., due to re-opening of a valve in the AVS module following recovery from a vacuum surge. Such a pressure pulse arrives at protection valve 100 from the aspiration-pump side of aspiration line 46 (from left to right according to the layout of Fig. 2).

Assuming piston 120 is in the "closed" position at the time the pressure pulse hits the valve, the pressure pulse exerts considerable force on the piston along the longitudinal axis of aspiration line 46 (left-to-right in the figure, perpendicularly to the axis of movement of the piston 120 in chamber 128). This force presses piston 120 against inlet 132. As a result, seal 130 compresses, and hermetically seals any remaining gap between piston 120 and inlet 132. The pressure pulse is thus effectively blocked from reaching the eye side of aspiration line 46.

In various examples, compressible seal 130 may be implemented from various materials and in various ways. The material composition of compressible seal 130 may comprise, for example, silicon, rubber, or any other suitable material that is sufficiently compressible and provides sufficient sealing when compressed. In one example, seal 130 may be fabricated by overmolding sealing material over the circumference of inlet 132. Alternatively, compressible seal 130 may comprise an O-ring that is placed over the circumference of inlet 132. Alternatively, any other suitable configuration can be used for implementing compressible seal 130.

A graph 110 at the bottom-left of Fig. 2 shows example pressure waveforms during restoration of vacuum level from a vacuum surge level 113 to a nominal vacuum level 111. A curve 115 illustrates a pressure pulse, which is potentially harmful to the eye, impinging on protection valve 100 from the aspiration-pump side. A typical nominal vacuum level (level 111) is 350 mmHg, whereas a typical peak pressure amplitude of pulse 115 may reach 650 mmHg. A curve 117 illustrates a dampened pressure pulse as may be reflected to the eye side of protection valve 100 with compressible seal 130.

The configuration of protection valve 100, as seen in Fig. 2, is an example configuration that is chosen purely for the sake of conceptual clarity. In alternative examples, any other suitable configuration can be used. For example, protection valve 100 may be inserted at any other suitable location in aspiration line 46, inside or outside handpiece 12. As another example, the axis of movement of piston 120 need not necessarily be perpendicular to the axis of aspiration line 46 (and thus to the direction of the force exerted by the pressure pulse). The two axes are typically not parallel with one another, but not necessarily perpendicular.

Fig. 3 is a flow chart that schematically illustrates a method for phacoemulsification, in accordance with an example of the present disclosure. The method begins with system 10 operating normally in performing a phacoemulsification procedure, at a normal operation stage 140. During normal operation, physician 15 operates handpiece 12 in the eye.

At a vacuum surge checking stage 144, processor 38 checks for occurrence of a post-occlusion vacuum surge. Such a vacuum surge may occur when a particle blocks the tip of needle 16 and is then removed (e.g., aspired). If no post-occlusion vacuum surge is detected, the method loops back to stage 140 above.

In response to detecting a post-occlusion vacuum surge, processor 38 closes aspiration line 46 and, subsequently releases the vacuum buildup in the aspiration line. Processor 38 may perform these actions, for example, using an anti-vacuum surge (AVS) module.

At a pressure-pulse checking stage 152, processor 38 checks whether the releasing of the vacuum buildup (at stage 148) is causing a distally-propagating pressure pulse. Processor 38 may check for occurrence of a pressure pulse using any suitable means, e.g., by reading a pressure sensor (not shown in the figure) that is coupled with the aspiration line proximally to protection valve 100. In absence of a pressure pulse, the method loops back to stage 140 above. (In some examples, stage 152 can be omitted, in which case the assumption is that every post-occlusion vacuum surge should be treated as potentially causing a distally-propagating pressure pulse.)

In response to detecting that a distally-propagating pressure pulse is imminent, processor 38 closes protection valve 100, at a valve closing stage 156. This action should be performed with small latency, so that piston 120 is guaranteed to be in the "closed" position when the pressure pulse hits the protection valve 100.

At a sealing stage 160, the pressure pulse hits, and presses piston 120 against compressible seal 130. Due to the pressure exerted by the pressure pulse, compressible seal 130 compresses between piston 120 and inlet 132, thereby sealing protection valve 100 hermetically.

After the pressure pulse dies down, at a valve opening step 164, processor 38 opens protection valve 100. The method loops back to stage 140 above, resuming normal operation.

The method flow of Fig. 3 is an example flow that is depicted purely for the sake of conceptual clarity. In alternative examples, any other suitable flow can be used.

Although the examples described herein mainly address mitigation of pressure pulses caused by recovery from post-occlusion vacuum surges, the methods and systems described herein can also be used in other applications, such as in mitigating pressure pulses caused by a peristaltic aspiration pump.

It will thus be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art

## Claims

1. A phacoemulsification system (10), comprising:
a hollow needle (16), which is configured to be inserted into an eye and to be vibrated so as to emulsify a lens of the eye;
an aspiration line (46), which is coupled with the hollow needle (16) for evacuating material from the eye; and
a protection valve (100) inserted in the aspiration line, the protection valve comprising:
a chamber (128) having (i) an inlet (132) for receiving the evacuated material arriving from the hollow needle (16) and (ii) an outlet (136) for flowing the material along the aspiration line (46);
a piston (120), configured to controllably move in the chamber (128) between at least (i) a first position that enables flow of the material between the inlet (132) and the outlet (136) and (ii) a second position that blocks the flow between the inlet (132) and the outlet (136); and **characterized by**
a seal (130), which is coupled with the inlet (132) and is configured, when the piston (120) is in the second position, to compress between the piston (120) and the inlet (132) in response to a pressure pulse that propagates in the aspiration line (46), thereby hermetically sealing the inlet (132).

2. The system (10) according to claim 1, wherein the seal (130) comprises a sealing material overmolded over a circumference of the inlet (132).

3. The system (10) according to claim 1, wherein the seal (130) comprises an O-ring placed over a circumference of the inlet (132).

4. The system (10) according to claim 1, wherein the protection valve (100) further comprises an electromagnet (124), configured to move the piston (120) between the first and second positions in response to a control signal.

5. The system (10) according to claim 1, and comprising a processor (38), which is configured to determine that the pressure pulse is imminent, and to control the protection valve (100) to close the aspiration line (46) before the pressure pulse impinges on the protection valve (100).

6. The system (10) according to claim 1, wherein the piston (120) is configured to move in the chamber (128) along a first axis, and wherein the seal (132) is configured to compress along a second axis that is not parallel with the first axis.

7. The system (10) according to claim 6, wherein the second axis is perpendicular to the first axis.

8. The system (10) according to claim 1, wherein the protection valve (100) is integrated in a phacoemulsification handpiece (12) that comprises the needle (16).

9. The system according to claim 1, wherein the protection valve (100) is inserted in the aspiration line (46) externally to a phacoemulsification handpiece (12) that comprises the needle (16).

10. A method for producing a phacoemulsification system (10), the method comprising:
providing a hollow needle (16), which is configured to be inserted into an eye and to be vibrated so as to emulsify a lens of the eye;
coupling an aspiration line (46) with the hollow needle (16) for evacuating material from the eye; and
inserting a protection valve (100) in the aspiration line (46), the protection valve (100) comprising:
a chamber (128) having (i) an inlet (132) for receiving the evacuated material arriving from the hollow needle (16) and (ii) an outlet (136) for flowing the material along the aspiration line (46);
a piston (120), configured to controllably move in the chamber (128) between at least (i) a first position that enables flow of the material between the inlet (132) and the outlet (136) and (ii) a second position that blocks the flow between the inlet (132) and the outlet (136); and
a seal (130), which is coupled with the inlet (132) and is configured, when the piston (120) is in the second position, to compress between the piston (120) and the inlet (132) in response to a pressure pulse that propagates in the aspiration line (46), thereby hermetically sealing the inlet (132).

## Patentansprüche

1. Phakoemulsifikationssystem (10), umfassend:
eine Hohlnadel (16), die konfiguriert ist, um in ein Auge eingeführt zu werden und in Vibration versetzt zu werden, um eine Linse des Auges zu emulgieren;
eine Absaugleitung (46), die mit der Hohlnadel (16) gekoppelt ist, um Material aus dem Auge zu entfernen; und
ein Schutzventil (100), das in die Absaugleitung eingesetzt ist, wobei das Schutzventil Folgendes umfasst:
eine Kammer (128) aufweisend (i) einen Einlass (132) zum Aufnehmen des entfernten Materials, das von der Hohlnadel (16) ankommt, und (ii) einen Auslass (136) zum Leiten des Materials entlang der Absaugleitung (46);
ein Kolben (120), der konfiguriert ist, um sich steuerbar in der Kammer (128) zwischen mindestens (i) einer ersten Position, die einen Fluss des Materials zwischen dem Einlass (132) und dem Auslass (136) ermöglicht, und (ii) einer zweiten Position, die den Fluss zwischen dem Einlass (132) und dem Auslass (136) blockiert, zu bewegen; und **gekennzeichnet durch**
eine Dichtung (130), die mit dem Einlass (132) gekoppelt ist und konfiguriert ist, um, wenn sich der Kolben (120) in der zweiten Position befindet, als Reaktion auf einen Druckimpuls, der sich in der Absaugleitung (46) ausbreitet, zwischen dem Kolben (120) und dem Einlass (132) komprimiert zu werden, wodurch der Einlass (132) hermetisch abgedichtet wird.

2. System (10) nach Anspruch 1, wobei die Dichtung (130) einen Dichtstoff umfasst, der über einen Umfang des Einlasses (132) umspritzt ist.

3. System (10) nach Anspruch 1, wobei die Dichtung (130) einen über einem Umfang des Einlasses (132) platzierten O-Ring umfasst.

4. System (10) nach Anspruch 1, wobei das Schutzventil (100) ferner einen Elektromagneten (124) umfasst, der konfiguriert ist, um den Kolben (120) als Reaktion auf ein Steuersignal zwischen der ersten und der zweiten Position zu bewegen.

5. System (10) nach Anspruch 1, und umfassend einen Prozessor (38), der konfiguriert ist, um zu bestimmen, dass der Druckimpuls unmittelbar bevorsteht, und um das Schutzventil (100) so zu steuern, dass es die Absaugleitung (46) schließt, bevor der Druckimpuls auf das Schutzventil (100) auftrifft.

6. System (10) nach Anspruch 1, wobei der Kolben (120) konfiguriert ist, um sich in der Kammer (128) entlang einer ersten Achse zu bewegen, und wobei die Dichtung (132) konfiguriert ist, um entlang einer zweiten Achse komprimiert zu werden, die nicht parallel zu der ersten Achse ist.

7. System (10) nach Anspruch 6, wobei die zweite Achse senkrecht zu der ersten Achse ist.

8. System (10) nach Anspruch 1, wobei das Schutzventil (100) in ein Phakoemulsifikationshandstück (12) integriert ist, das die Nadel (16) umfasst.

9. System nach Anspruch 1, wobei das Schutzventil (100) in die Absaugleitung (46) außerhalb eines Phakoemulsifikationshandstücks (12) eingesetzt ist, das die Nadel (16) umfasst.

10. Verfahren zum Produzieren eines Phakoemulsifikationssystems (10), das Verfahren umfassend:
Bereitstellen einer Hohlnadel (16), die konfiguriert ist, um in ein Auge eingeführt zu werden und in Vibration versetzt zu werden, um eine Linse des Auges zu emulgieren;
Koppeln einer Absaugleitung (46) mit der Hohlnadel (16) zum Entfernen von Material aus dem Auge; und
Einsetzen eines Schutzventils (100) in die Absaugleitung (46), wobei das Schutzventil (100) Folgendes umfasst:
eine Kammer (128) aufweisend (i) einen Einlass (132) zum Aufnehmen des entfernten Materials, das von der Hohlnadel (16) ankommt, und (ii) einen Auslass (136) zum Leiten des Materials entlang der Absaugleitung (46);
ein Kolben (120), der konfiguriert ist, um sich steuerbar in der Kammer (128) zwischen mindestens (i) einer ersten Position, die einen Fluss des Materials zwischen dem Einlass (132) und dem Auslass (136) ermöglicht, und (ii) einer zweiten Position, die den Fluss zwischen dem Einlass (132) und dem Auslass (136) blockiert, zu bewegen; und
eine Dichtung (130), die mit dem Einlass (132) gekoppelt ist und konfiguriert ist, um, wenn sich der Kolben (120) in der zweiten Position befindet, als Reaktion auf einen Druckimpuls, der sich in der Absaugleitung (46) ausbreitet, zwischen dem Kolben (120) und dem Einlass (132) komprimiert zu werden, wodurch der Einlass (132) hermetisch abgedichtet wird.

## Revendications

1. Système de phacoémulsification (10), comprenant :
une aiguille creuse (16), qui est conçue pour être insérée dans un œil et pour être mise en vibration de façon à émulsifier un cristallin de l'œil ;
une ligne d'aspiration (46), qui est accouplée à l'aiguille creuse (16) pour l'évacuation de matériau hors de l'œil ; et
une valve de protection (100) insérée dans la ligne d'aspiration, la valve de protection comprenant :
une chambre (128) ayant (i) une entrée (132) permettant de recevoir le matériau évacué arrivant de l'aiguille creuse (16) et (ii) une sortie (136) permettant l'écoulement du matériau le long de la ligne d'aspiration (46) ;
un piston (120), conçu pour se déplacer de manière commandée dans la chambre (128) entre au moins (i) une première position qui permet un écoulement du matériau entre l'entrée (132) et la sortie (136) et (ii) une seconde position qui bloque l'écoulement entre l'entrée (132) et la sortie (136) ; et **caractérisé par**
un joint d'étanchéité (130), qui est accouplé à l'entrée (132) et est conçu, lorsque le piston (120) est dans la seconde position, pour se comprimer entre le piston (120) et l'entrée (132) en réponse à une impulsion de pression qui se propage dans la ligne d'aspiration (46), assurant de ce fait l'étanchéité hermétique de l'entrée (132).

2. Système (10) selon la revendication 1, dans lequel le joint d'étanchéité (130) comprend un matériau d'étanchéité surmoulé par-dessus une circonférence de l'entrée (132).

3. Système (10) selon la revendication 1, dans lequel le joint d'étanchéité (130) comprend un joint torique placé par-dessus une circonférence de l'entrée (132).

4. Système (10) selon la revendication 1, dans lequel la valve de protection (100) comprend en outre un électroaimant (124), conçu pour déplacer le piston (120) entre les première et seconde positions en réponse à un signal de commande.

5. Système (10) selon la revendication 1, et comprenant un processeur (38), qui est configuré pour déterminer que l'impulsion de pression est imminente, et pour commander la valve de protection (100) pour fermer la ligne d'aspiration (46) avant que l'impulsion de pression ne touche la valve de protection (100).

6. Système (10) selon la revendication 1, dans lequel le piston (120) est conçu pour se déplacer dans la chambre (128) le long d'un premier axe, et dans lequel le joint d'étanchéité (132) est conçu pour se comprimer le long d'un second axe qui n'est pas parallèle au premier axe.

7. Système (10) selon la revendication 6, dans lequel le second axe est perpendiculaire au premier axe.

8. Système (10) selon la revendication 1, dans lequel la valve de protection (100) est intégrée dans une pièce à main de phacoémulsification (12) qui comprend l'aiguille (16).

9. Système selon la revendication 1, dans lequel la valve de protection (100) est insérée dans la ligne d'aspiration (46) de façon externe à une pièce à main de phacoémulsification (12) qui comprend l'aiguille (16).

10. Procédé permettant de produire un système de phacoémulsification (10), le procédé comprenant :
la fourniture d'une aiguille creuse (16), qui est conçue pour être insérée dans un œil et pour être mise en vibration de façon à émulsifier un cristallin de l'œil ;
l'accouplement d'une ligne d'aspiration (46) à l'aiguille creuse (16) pour l'évacuation de matériau hors de l'œil ; et
l'insertion d'une valve de protection (100) dans la ligne d'aspiration (46), la valve de protection (100) comprenant :
une chambre (128) ayant (i) une entrée (132) permettant de recevoir le matériau évacué arrivant de l'aiguille creuse (16) et (ii) une sortie (136) permettant l'écoulement du matériau le long de la ligne d'aspiration (46) ;
un piston (120), conçu pour se déplacer de manière commandée dans la chambre (128) entre au moins (i) une première position qui permet un écoulement du matériau entre l'entrée (132) et la sortie (136) et (ii) une seconde position qui bloque l'écoulement entre l'entrée (132) et la sortie (136) ; et
un joint d'étanchéité (130), qui est accouplé à l'entrée (132) et est conçu, lorsque le piston (120) est dans la seconde position, pour se comprimer entre le piston (120) et l'entrée (132) en réponse à une impulsion de pression qui se propage dans la ligne d'aspiration (46), assurant de ce fait l'étanchéité hermétique de l'entrée (132).
